Europäisches Patentamt

European Patent Office

Office européen des brevets

(11) Veröffentlichungsnummer: **0 435 072 B1**

(12) **EUROPÄISCHE PATENTSCHRIFT**

(45) Veröffentlichungstag der Patentschrift: **27.04.94**

(21) Anmeldenummer: **90123912.9**

(22) Anmeldetag: **12.12.90**

(51) Int. Cl.⁵: **C07C 209/26**, C07C 211/08

(54) **Verfahren zur Herstellung von N,N-Dimethylaminen.**

(30) Priorität: **23.12.89 DE 3942793**

(43) Veröffentlichungstag der Anmeldung:
**03.07.91 Patentblatt 91/27**

(45) Bekanntmachung des Hinweises auf die
Patenterteilung:
**27.04.94 Patentblatt 94/17**

(84) Benannte Vertragsstaaten:
**AT BE CH DE ES FR GB IT LI NL SE**

(56) Entgegenhaltungen:
**DE-A- 2 907 869**
**DE-A- 3 721 539**
**DE-B- 2 535 073**

(73) Patentinhaber: **HOECHST AKTIENGESELL-SCHAFT**

**D-65926 Frankfurt(DE)**

(72) Erfinder: **Kampmann, Detlef, Dr. Dipl.-Chem.**
**Friedhofstrasse 100**
**W-4630 Bochum 6(DE)**
Erfinder: **Lukas, Rainer, Dr. Dipl.-Chem.**
**Holunderweg 13**
**W-4300 Essen 1(DE)**
Erfinder: **Kniep, Claus**
**Rosenstrasse 93**
**W-4200 Oberhausen 1(DE)**

**Beschreibung**

Die vorliegende Erfindung betrifft ein Verfahren zur Herstellung von N,N-Dimethylaminen durch Umsetzung eines Aldehyds mit Dimethylamin und Wasserstoff unter Druck und bei erhöhter Temperatur in Gegenwart eines Hydrierkatalysators.

Die Reaktion führt zu einem Austausch des Sauerstoffatoms in der Aldehydgruppe durch einen Dimethylaminrest, wobei Wasser gebildet wird. Hierbei kann als Zwischenstufe ein Enamin gebildet werden, das mittels Wasserstoff in das entsprechende Dimethylaminderivat überführt wird. Unter N,N-Dimethylaminen werden im folgenden tertiäre Amine, die neben zwei Methylgruppen noch einen beliebigen Rest, der auf den Einbau des Aldehyds zurückzuführen ist, verstanden. Tertiäre Amine stellen technisch bedeutsame Verbindungen dar. Sie können als Polymerisations- und Härtungskatalysatoren für die Herstellung von Kunststoffen auf Epoxid- und Urethanbasis dienen. Ferner sind sie als Korrosionsinhibitoren und Absorptionsmittel für die Synthesegaswäsche geeignet. Dies trifft in besonderem Maße auf die leicht herstellbaren N,N-Dimethylamine zu.

Die US-PS 4 207 260 beschreibt die Herstellung tertiärer Amine durch Umsetzung eines Aldehyds mit Wasserstoff und einem Amin in Gegenwart eines Rhodium- oder Rutheniumcarbonyls oder -halogencarbonyls als Katalysator.

Die US-PS 4 229 374 betrifft ein Verfahren zur Herstellung eines Amins durch Umsetzung eines Alkohols, Aldehyds oder Ketons mit Ammoniak, einem primären oder sekundären Amin in einer reduzierenden Atmosphäre mittels eines Kupfer, Zinn und gegebenenfalls ein Alkali- oder Erdalkalimetall enthaltenden Träger-Katalysators.

Die Umsetzung von Aldehyden mit Dimethylamin und Wasserstoff - auch unter dem Begriff reduktive Aminierung von Aldehyden bekannt - ist ein technisch gangbarer Weg zur Herstellung von N,N-Dimethylaminen.

Hinweise bezüglich dieses Verfahrens finden sich in Houben-Weyl, Methoden der organischen Chemie, Band IV/Ic Seiten 423 bis 425, 4. Auflage (1980), Georg Thieme Verlag Stuttgart-New York und bei S. B. Cavitt et al., J. Org. Chem. 27, 1211 (1962).

Die Umsetzung läßt sich absatzweise oder kontinuierlich durchführen. Bei kontinuierlicher Verfahrensweise verwendet man üblicherweise druckfeste Rohrreaktionen, die den Hydrierkatalysator in stückiger Form enthalten. Die Ausgangsstoffe Dimethylamin, Aldehyd und Wasserstoff werden dem Rohrreaktor entweder am Boden oder am Kopf zugeführt. Je nach Art der Zugabe spricht man von Riesel- oder Sumpffahrweise.

Es ist allerdings auch möglich, die Reaktion in Gegenwart eines suspendierten Hydrierkatalysators sowohl diskontinuierlich als auch kontinuierlich vorzunehmen.

Im Verlauf der Umsetzung treten verschiedene Nebenprodukte auf. Besonders störend wirkt sich vor allem die Bildung sekundärer N-Methylamine, die möglicherweise aufgrund einer Spaltung von Dimethylamin und bereits gebildeten N,N-Dimethylaminen entstehen, aus. Diese sekundären N-Methylamine lassen sich von dem gewünschten Wertprodukt, nämlich von den N,N-Dimethylaminen, wegen der eng beieinander liegenden Siedepunkte destillativ nicht im erforderlichen Umfange abtrennen.

Für eine Reihe von Anwendungsgebieten werden an die Reinheit der einzusetzenden N,N-Dimethylamine recht hohe Anforderungen gestellt. Da in diesen Fällen bereits geringe Mengen sekundärer N-Methylamine eine Verwendung der N,N-Dimethylamine verhindern, bestand ein Bedarf nach einem Verfahren, das sowohl einfach auszuüben ist als auch den Gebrauch üblicher Hydrierkatalysatoren ermöglicht und zugleich nicht nur die vorstehend genannten, auf der Anwesenheit sekundärer N-Methylamine beruhenden Nachteile umgeht, sondern auch das erwünschte Wertprodukt in hoher Ausbeute liefert.

Diese Aufgaben werden gelöst durch ein Verfahren zur Herstellung von N,N-Dimethylaminen durch Umsetzung eines Aldehyds mit Dimethylamin und Wasserstoff unter Druck und bei erhöhter Temperatur in Gegenwart eines Hydrierkatalysators. Es ist dadurch gekennzeichnet, daß man nicht umgesetztes Dimethylamin abtrennt, anschließend 0,1 bis 25 Gew.-% Formaldehyd oder Formaldehyd bildende Substanz bezogen auf N,N-Dimethylamin zusetzt und destilliert.

Das erfindungsgemäße Verfahren läßt sich - wie eingangs beschrieben - unter Verwendung eines in feinverteiltem Zustand suspendierten oder in stückiger Form vorliegenden Hydrierkatalysators sowohl diskontinuierlich als auch kontinuierlich betreiben. Es ist besonders für einen kontinuierlichen Prozeß geeignet.

Arbeitet man mit einem suspendierten Hydrierkatalysator, so wird man die Katalysatorsuspension beispielsweise in einem Rührbehälter verlegen und die Einsatzstoffe in die gerührte Suspension einbringen.

Verwendet man einen stückigen Hydrierkatalysator, so empfiehlt es sich, diesen in Form einer Schüttung in einem druckfesten Reaktionsrohr anzuordnen und die Ausgangsstoffe über diese Schüttung zu führen. Die Einsatzstoffe Aldehyd und Dimethylamin können sowohl im gasförmigen als auch im flüssigen Zustand in die Reaktion eingesetzt werden. Besonders einfach gestaltet sich das erfindungsgemäße Verfahren bei Ausübung der Umsetzung in flüssiger Phase.

Als Aldehyde kommen beliebige aromatische, araliphatische, cycloaliphatische und aliphatische, insbesondere araliphatische, cycloaliphatische und aliphatische, bevorzugt cycloaliphatische und aliphatische, besonders bevorzugt aliphatische Aldehyde und deren Derivate in Betracht.

Stellvertretend für aromatische Aldehyde sind Benzaldehyd, p-Hydroxybenzaldehyd, für araliphatische Aldehyde, Phenylacetaldehyd, Phenylpropionaldehyd, für cycloaliphatische Aldehyde Formylcyclohexan und für aliphatische Aldehyde geradkettige und verzweigte Aldehyde mit 2 bis 20, insbesondere 2 bis 15, bevorzugt 2 bis 10 Kohlenstoffatomen zu nennen. Beispiele für geeignete aliphatische Aldehyde sind Propanal, n- und i-Butanal, n- und i-Pentanal, wie 3-Methylbutanal, n- und i-Hexanal, Heptanale, Octanale, beispielsweise 2-Ethylhexanal, Nonanale, Decanale, Undecanale, Dodecanale, Tridecanale, Aldehyde mit 14 bis 20 Kohlenstoffatomen, wie Hexadecanale, aber auch ungesättigte Aldehyde wie Acrolein, Crotonaldehyd, Pentenale, Hexenale, Octenale wie 2-Ethylhexenal.

Der Aldehyd kann sowohl in verdünnter Form als auch in reinem Zustand eingesetzt werden. In einer Vielzahl von Fällen kann vorteilhaft auf den Gebrauch eines Lösungsmittels verzichtet und der Aldehyd in unverdünnter Form verwendet werden.

Das für die Umsetzung benötigte Dimethylamin kann sowohl in gelöster Form als auch als unverdünnter Stoff eingesetzt werden. Als Lösungsmittel eignet sich Wasser. Es ist jedoch ohne weiteres möglich, reines Dimethylamin in unverdünntem Zustand für die Umsetzung zu gebrauchen. Wegen seines niedrigen Siedepunktes ist es in geeigneten Druckbehältern aufzubewahren.

Aldehyd und Dimethylamin können vor Einsatz in die Reaktion zusammengeführt und als Gemisch in die Reaktion eingesetzt werden. Beide Einsatzstoffe können aber auch voneinander getrennt in die Umsetzung eingeführt werden. Den für die Reaktion benötigten Wasserstoff speist man üblicherweise über eine separate Leitung, die gegebenenfalls Vorrichtungen zur gleichmäßigen Verteilung des Wasserstoffs aufweist, in das den Hydrierkatalysator aufweisende Reaktionsgefäß.

Man setzt den Aldehyd mit Dimethylamin und Wasserstoff im Molverhältnis 1 : (1 bis 10) : (1 bis 50), insbesondere 1 : (1 bis 8) : (1 bis 30), bevorzugt 1 : (1 bis 5) : (1 bis 10) ein. Es hat sich bewährt, Dimethylamin und Wasserstoff im Überschuß bezogen auf Aldehyd einzusetzen.

Im allgemeinen reicht eine über den stöchiometrischen Bedarf hinausgehende Menge von 50 Mol % Dimethylamin und 100 Mol % Wasserstoff. Die Umsetzung erfolgt bei 1 bis 20, insbesondere 3 bis 15, bevorzugt 7 bis 12 MPa und 50 bis 150, insbesondere 70 bis 130, bevorzugt 100 bis 110°C.

Als Hydrierkatalysatoren können übliche Hydrierkontakte verwendet werden. Druck und Temperatur sind im gewissen Umfange auch von der Art des Hydrierkatalysators abhängig und müssen demzufolge entsprechend aufeinander abgestimmt werden.

Der Hydrierkatalysator kann ein trägerfreier Metallkatalysator oder ein Trägerkatalysator sein, der als Träger $Al_2O_3$, $SiO_2$, Kieselerde, Aktivkohle oder Bimsstein, insbesondere $SiO_2$, Kieselerde oder Aktivkohle, bevorzugt $SiO_2$, Kieselerde enthält. Der Hydrierkatalysator enthält Ni, Co, Cu, Mn, Fe, Rh, Pd und/oder Pt, insbesondere Ni, Co, Cu, Rh, Pd und/oder Pt, bevorzugt Ni, Co und/oder Rh, besonders bevorzugt Ni und daneben gegebenenfalls gebräuchliche Zusatzstoffe und Promotoren, wie Erdalkalimetalloxide, $SiO_2$, $Al_2O_3$, $MnO_2$ und/oder $Cr_2O_3$.

Als trägerfreie Katalysatoren können Raney-Katalysatoren wie Raney-Nickel oder Raney-Cobalt, aber auch solche auf Basis von Palladium, Rhodium oder Platin eingesetzt werden.

Besonders einfach gestaltet sich das erfindungsgemäße Verfahren bei Gebrauch trägerhaltiger Hydrierkatalysatoren mit 10 bis 75, insbesondere 20 bis 70, bevorzugt 40 bis 65 Gew.-% Ni, Co, Cu, Mn und/oder Fe, insbesondere Ni, Co, Cu und/oder Mn, bevorzugt Ni, Co und/oder Cu bezogen auf gesamte Katalysatormasse.

Edelmetallkatalysatoren gestatten eine Umsetzung unter besonders milden Bedingungen. Sie sind üblicherweise auf einem Träger untergebracht und weisen einen Metallgehalt von 0,1 bis 20, insbesondere 0,2 bis 15, bevorzugt 0,5 bis 10 Gew.-% Pd, Rh und/oder Pt bezogen auf gesamte Katalysatormasse auf. Als Träger empfehlen sich geformte Materialien auf Basis von $Al_2O_3$, $SiO_2$, Kieselerde, Aktivkohle und/oder Bimsstein.

Der Katalysator bestimmt die Reaktionsbedingungen, insbesondere die Reaktionstemperatur und den Druck. Üblicherweise läßt sich die Umsetzung bei 50 bis 150, insbesondere 70 bis 130, bevorzugt 100 bis 110°C und 1 bis 20, insbesondere 3 bis 15, bevorzugt 7 bis 12 MPa mit gutem Erfolg durchführen.

Gebraucht man einen stückigen Hydrierkatalysator, so kann man die Umsetzung sowohl im geraden Durchgang als auch unter Zuhilfenahme eines Reaktionsproduktkreislaufes ablaufen lassen. Die Ausgangsstoffe werden dem Reaktor - üblicherweise einem senkrecht angeordneten Rohrreaktor - worin der Katalysator als Schüttschicht untergebracht ist, am Kopf oder am Boden zugeleitet. Nach einer besonderen Ausführungsform werden die Einsatzstoffe dem Reaktor am Boden zugeführt und die Umsetzung erfolgt im geraden Durchgang, d. h. ohne Kreislauf des Reaktionsproduktes. Das Reaktionsgemisch verläßt den Reaktor über Kopf. In einem dem Reaktor nachgeschalteten Abscheider wird Wasserstoff zusammen mit gegebenenfalls nicht umgesetztem Dimethylamin unter Überdruck aus dem Reaktionsgemisch entfernt. Unverbrauchter Wasserstoff und nicht umgesetztes Dimethylamin können in die Reaktion wieder eingesetzt werden.

Für das Gelingen des erfindungsgemäßen Verfahrens ist es wichtig, noch vorhandenes Dimethylamin aus dem Reaktionsgemisch gründlich zu entfernen. Üblicherweise führt man diese Abtrennung in zwei oder mehreren Stufen durch, wobei in der ersten Stufe unter einem Druck von 0,5 bis 1,0 MPa und in den nachfolgenden Stufen bei 0,05 bis 0,15 MPa destilliert wird. In den meisten Fällen genügt eine zweistufige Abtrennung des Dimethylamins.

Formaldehyd kann man sowohl in reiner Form als auch in Lösung einsetzen. Geeignete Lösungsmittel sind aliphatische Alkohole mit 1 bis 4 Kohlenstoffatomen und/oder Wasser. Besonders einfach läßt sich das erfindungsgemäße Verfahren unter Verwendung wäßriger Lösungen, die beispielsweise 10 bis 60, insbesondere 15 bis 55, bevorzugt 20 bis 50 Gew.-% Formaldehyd aufweisen, ausüben.

Als Formaldehyd bildende Substanz bietet sich Paraformaldehyd als Reinprodukt oder in suspendierter oder gelöster Form an. Brauchbare Solventien zur Herstellung von Paraformaldehyd-Suspensionen sind Wasser, Methanol und Ethanol. Will man eine Paraformaldehydlösung gebrauchen, so empfehlen sich Methanol und Wasser, insbesondere Wasser als Solvens. Den Formaldehyd setzt man dem Reaktionsgemisch ebenso wie die Formaldehyd bildende Substanz erst nach Abtrennung des Dimethylamins zu.

Eine Menge von 1 bis 10, insbesondere 1,1 bis 5, bevorzugt 1,5 bis 2 Gew.-% Formaldehyd oder Formaldehyd bildende Substanz bezogen jeweils auf N,N-Dimethylamin reicht in der Regel aus, um den gewünschten Erfolg sicherzustellen.

Man läßt den Formaldehyd unter einem Druck von 0,05 bis 1,0 MPa, insbesondere 0,1 bis 0,5 MPa und bei 50 bis 250, insbesondere 70 bis 200, bevorzugt 80 bis 180 °C auf das Reaktionsprodukt einwirken. Sehr einfach gestaltet sich dies, wenn man den Formaldehyd oder die Formaldehyd bildende Substanz nach Abtrennung des Dimethylamins dem Reaktionsprodukt beispielsweise vor Einsatz in die Destillation oder bevorzugt unmittelbar in den Sumpf der ersten Destillationskolonne zuleitet.

Im allgemeinen genügen die im Sumpf der Kolonne vorliegenden Bedingungen, die unerwünschten sekundären N-Methylamine zu beseitigen. Möglicherweise ist hierfür eine Reaktion zwischen dem sekundären N-Methylamin und dem Formaldehyd verantwortlich, die überraschenderweise zu einem Produkt führt, das sich ohne Schwierigkeiten reinigen läßt.

Ist der Anteil sekundärer N-Methylamine im Reaktionsprodukt bekannt, so kann man die Formaldehydzugabe an diesem Wert ausrichten. Es empfiehlt sich, Formaldehyd in ausreichendem Überschuß bezogen auf sekundäres N-Methylamin zu gebrauchen. Üblicherweise fügt man je Mol sekundäres N-Methylamin 1 bis 10, insbesondere 1,2 bis 5 Mol, bevorzugt 1,5 bis 2 Mol Formaldehyd zu. Auf diese Weise lassen sich nicht nur größere Anteile (>2 Gew.-%), sondern auch recht geringe Mengen, beispielsweise 2 Gew.-% und weniger sekundäres N-Methylamin, nahezu restlos entfernen.

Zur Beseitigung des bei der N,N-Dimethylamin-Synthese gebildeten Wassers kann man dem Reaktionsgemisch ein organisches Lösungsmittel als Schleppmittel zusetzen. Geeignete Solventien sind aliphatische Kohlenwasserstoffe, Hexen, Cyclohexan, Benzol , Toluol, insbesondere Hexen, Cyclohexan, bevorzugt Hexen.

Die destillative Abtrennung des Wassers erfolgt in einer oder zweckmäßigerweise zwei oder mehreren Stufen. Die Auswahl des organischen Lösungsmittels richtet sich ebenso wie die Anzahl der Destillationsschritte auch nach der Art des N,N-Dimethylamins. Die Gewinnung von reinem N,N-Dimethylamin mit einem Gehalt >99, insbesondere >99,5 Gew.-% mittels fraktionierter Destillation bereitet üblicherweise keine Schwierigkeiten. Unter Verwendung einer Kolonne mit 20 bis 30 theoretischen Böden fällt das N,N-Dimethylamin-Reinprodukt als 4. oder 5. Fraktion an. Besonders günstig gestaltet sich das Entfernen des Wassers, wenn man das heterogen anfallende Wasser aus dem als Kopfprodukt abgenommenen Destillat abtrennt und das Schleppmittel im Kreislauf in die Kolonne zurückführt.

Formaldehyd oder eine Formaldehyd bildende Substanz läßt sich allgemein zur Entfernung von sekundären N-Methylaminen aus sekundäre N-Methylamine und N,N-Dimethylamine enthaltenden Gemischen verwenden. Man fügt den Formaldehyd oder die Formaldehyd bildende Substanz hinzu und erwärmt unter Durchmischung auf 50 bis 250 °C. Bereits nach kurzer Zeit hat der Gehalt an sekundärem N-Methylamin deutlich abgenommen. Wird eine weitere Verringerung gewünscht, so empfiehlt sich eine neuerliche Zugabe von Formaldehyd. Üblicherweise reicht eine Menge von 1,5 bis 5,0, insbesondere 1,5 bis 3,0, bevorzugt 1,5 bis 2,0 Mol Formaldehyd je Mol zu entfernendes sekundäres N-Methylamin aus, den Anteil an sekundärem N-Methylamin im erforderlichen Umfange herabzusetzen.

Das Reaktionsgemisch wird nach Behandlung mit Formaldehyd fraktioniert destilliert, wobei zunächst gegebenenfalls vorhandenes Wasser mit Hilfe eines geeigneten Schleppmittels destillativ abgetrennt wird. Nach Abtrennung von Vorlaufprodukten gelingt es zumeist, das N,N-Dimethylamin als 4. oder 5. Fraktion in einer Reinheit >99, insbesondere >99,5 Gew.-% zu gewinnen.

Die nachfolgend aufgeführten Beispiele belegen die Erfindung, ohne sie zu begrenzen.

Experimenteller Teil

Der Reaktorbehälter besteht aus einem druckfesten Rohr mit einem Innendurchmesser von 28 mm. In den unteren Teil des Druckrohres werden Raschigringe, die einen Durchmesser von 3 mm aufweisen bis zu einer Höhe von 800 mm aufgefüllt. Diese Zone dient als Vorheizzone, worin die Ausgangsstoffe auf die vorgegebene Temperatur aufgeheizt werden. Oberhalb dieser Vorheizzone befindet sich ein festangeordneter Hydrierkatalysator in Tablettenform. Mittels eines den Reaktor umgebenden Heizmantels wird die gewünschte Temperatur eingestellt.

Zu Beginn der Umsetzung wird der Reaktor mit N,N-Dimethylamin befüllt. Anschließend werden die Einsatzstoffe Aldehyd, Dimethylamin und Wasserstoff von unten in den Reaktor eingebracht, durchströmen die mit Raschigringen befüllte Vorheizzone und gelangen in die Katalysatorzone, wo die Umsetzung stattfindet. Nach Verlassen der Katalysatorzone wird das Reaktionsgemisch am Kopf des Reaktors abgezogen und in einen Druckabscheider überführt, in dem flüssiges Reaktionsprodukt von nicht umgesetztem Dimethylamin und Wasserstoff abgetrennt wird. Anschließend wird das Reaktionsgemisch einer für eine Destillation unter Überdruck geeigneten Kolonne zugeleitet und von restlichem Dimethylamin und gegebenenfalls Wasserstoff befreit. Das vom Sumpf dieser Kolonne abgezogene Reaktionsgemisch dient als Einsatzstoff für die fraktionierte Destillation. Der Formaldehyd wird dem während der destillativen Abtrennung des Vorlaufes anfallenden Sumpfprodukt zugesetzt. Die Zugabe des die Abtrennung von Wasser erleichternden Schleppmittels erfolgt anschließend, wobei zwei wasserhaltige Fraktionen gewonnen werden, bevor nach Abtrennung eines Zwischenlaufes das Wertprodukt abdestilliert wird.

Beispiel 1

Kontinuierliche Herstellung von N,N-Dimethyl-n-butylamin

In dem vorstehend beschriebenen Reaktorbehälter befinden sich 300 ml eines tablettierten Ni-Katalysators (~50 bis 53 Gew.-% Ni und etwa 25 bis 30 Gew.-% Kieselgur als Träger; Handelsprodukt der Hoechst AG: RCH Ni 52/35).

Der Reaktor wird mit N,N-Dimethyl-n-butylamin gefüllt. Anschließend werden bei 105 bis 110°C und 8 MPa $H_2$-Druck 56 ml n-Butanal/h, 200 ml Dimethylamin/h und 34 Nl Wasserstoff/h dem Reaktor von unten zugeleitet. Das entstehende Reaktionsgemisch wird über den Kopf des Reaktorbehälters abgezogen und in dem nachgeschalteten Druckabscheider, der unter dem gleichen Druck wie der Reaktor steht, von überschüssigem Dimethylamin und Wasserstoff abgetrennt.

Die nachfolgende Druckdestillation wird bei 0,7 MPa und etwa 130°C Sumpftemperatur betrieben, wobei restliches Dimethylamin und gegebenenfalls noch vorhandener Wasserstoff entfernt werden. Die Destillation erfolgt bei Normaldruck mittels einer Kolonne mit 30 theoretischen Böden.

Die erste Fraktion fällt bei 80°C Kopf- und etwa 93°C Sumpftemperatur an. In das Sumpfprodukt werden ~ 3 Gew.-% Formalinlösung (37 %ige wäßrige Lösung)/h bezogen auf N,N-Dimethyl-n-butylamin eingespeist.

Die zweite Fraktion wird bei 66°C Kopf- und 85°C Sumpftemperatur unter Zusatz von Hexen, die dritte Fraktion wird bei 75°C Kopf- und ∿ 95°C Sumpftemperatur ebenfalls unter Zusatz von Hexen, das als Schleppmittel zur Wasserentfernung dient, gewonnen. Das Wasser-Hexen-Gemisch wird getrennt, das Wasser abgezogen und das Hexen in die Kolonne zurückgeführt.

Die Zwischenlauffraktion geht bei ~ 90-92°C über Kopf, die Sumpftemperatur beträgt ca. 96-98°C. Das reine Wertprodukt wird bei 93°C Kopf- und 110-112°C Sumpftemperatur gewonnen. Die Zusammensetzung des Wertproduktes ist Tabelle 1 zu entnehmen.

Vergleichsversuch

Es wird wie in Beispiel 1 beschrieben gearbeitet, jedoch auf eine Zugabe von Formaldehyd verzichtet. Die Zusammensetzung des Wertproduktes ist Tabelle 1 zu entnehmen.

## Tabelle 1

| | Wertprodukt aus Beispiel 1 | Wertprodukt aus Vergleichs- versuch 1 |
|---|---|---|
| **Zusammensetzung** | | |
| Kohlenwasserstoff | 0,11 Gew.-% | 0,17 Gew.-% |
| Vorlauf | 0,14 Gew.-% | 0,15 Gew.-% |
| N,N-Dimethyl- n-butylamin | 99,65 Gew.-% | 98,09 Gew.-% |
| N-Methyl- n-butylamin | 0,02 Gew.-% | 1,22 Gew.-% |
| Nachlauf | 0,07 Gew.-% | 0,37 Gew.-% |

Wie aus obenstehender gaschromatografisch ermittelten Analyse (wasserfrei gerechnet) hervorgeht, hat der Zusatz von Formaldehyd eine sehr deutliche Verringerung des sek. N-Methylamins (hier N-Methyl-n-butylamin) bewirkt.

Beispiel 2

Verwendung von Formaldehyd zum Beseitigen von N-Methyl-n-butylamin

In einem 2 l Dreihalskolben, der mit einem Rührer, einem Thermometer und einem Rückflußkühler bestückt ist, werden 1000 g durch fraktionierte Destillation gewonnenes N,N-Dimethyl-n-butylamin (Gehalt 98,3 Gew.-%), das durch 1,5 Gew.-% destillativ nicht mehr abtrennbares N-Methyl-n-butylamin verunreinigt ist, vorgelegt. Anschließend werden 30 g Formalinlösung (37 Gew.-% Formaldehyd in Wasser) entsprechend 1,1 Gew.-% Formaldehyd bezogen auf Amin zugesetzt.

Man erwärmt unter Rühren bis auf Rückflußtemperatur, läßt 30 Minuten reagieren und kühlt ab. Das anfallende Produkt weist gemäß gaschromatischer Analyse (wasserfrei gerechnet) nunmehr 99,6 Gew.-% N,N-Dimethyl-n-butylamin auf. Sekundäres N-Methylamin (hier N-Methyl-n-butylamin) ist gaschromatografisch nicht mehr nachweisbar.

**Patentansprüche**

1. Verfahren zur Herstellung von N,N-Dimethylaminen durch Umsetzung eines Aldehyds mit Dimethylamin und Wasserstoff unter Druck und bei erhöhter Temperatur in Gegenwart eines Hydrierkatalysators, dadurch gekennzeichnet, daß man nicht umgesetztes Dimethylamin abtrennt, anschließend 0,1 bis 25 Gew.-% Formaldehyd oder Formaldehyd bildende Substanz bezogen auf N,N-Dimethylamin zusetzt und destilliert.

2. Verfahren nach Anspruch 1, dadurch gekennzeichnet, daß man den Aldehyd mit Dimethylamin und Wasserstoff in Mol-Verhältnis 1 : (1 bis 10) : (1 bis 50), insbesondere 1 : (1 bis 8) : (1 bis 30) bei 1 bis 20, insbesondere 3 bis 15 MPa und 50 bis 150, insbesondere 70 bis 130 °C umsetzt.

**3.** Verfahren nach Anspruch 1 oder 2, dadurch gekennzeichnet, daß der Hydrierkatalysator ein trägerfreier Metallkatalysator oder ein Trägerkatalysator ist, der als Träger $Al_2O_3$, $SiO_2$, Kieselerde, Aktivkohle oder Bimsstein, insbesondere $SiO_2$, Kieselerde oder Aktivkohle enthält.

**4.** Verfahren nach einem oder mehreren der Ansprüche 1 bis 3, dadurch gekennzeichnet, daß der Hydrierkatalysator Ni, Co, Cu, Mn, Fe, Rh, Pd und/oder Pt, insbesondere Ni, Co, Cu, Rh, Pd und/oder Pt und daneben gegebenenfalls gebräuchliche Zusatzstoffe und Promotoren, wie Erdalkalimetalloxide, $SiO_2$, $Al_2O_3$, $MnO_2$ und/oder $Cr_2O_3$ enthalten.

**5.** Verfahren nach einem oder mehreren der Ansprüche 1 bis 4, dadurch gekennzeichnet, daß man nicht umgesetztes Dimethylamin zunächst bei 0,5 bis 1,0 MPa und anschließend bei 0,05 bis 0,15 MPa destillativ abtrennt.

**6.** Verfahren nach einem oder mehreren der Ansprüche 1 bis 5, dadurch gekennzeichnet, daß man 1 bis 10, insbesondere 1,1 bis 5 Gew.-% Formaldehyd oder Formaldehyd bildende Substanz bezogen auf N,N-Dimethylamin zusetzt.

**7.** Verfahren nach einem oder mehreren der Ansprüche 1 bis 6, dadurch gekennzeichnet, daß man den Formaldehyd bei 0,05 bis 1,0, insbesondere 0,1 bis 0,5 MPa und 50 bis 250, insbesondere 70 bis 200 °C zusetzt.

**8.** Verfahren nach einem oder mehreren der Ansprüche 1 bis 7, dadurch gekennzeichnet, daß man in Gegenwart eines wasserschleppenden Lösungsmittel und gegebenenfalls in mehreren Stufen destilliert.

**9.** Verwendung von Formaldehyd oder einer Formaldehyd bildenden Substanz zur Entfernung von sekundären N-Methylaminen aus sekundäre N-Methylamine und N,N-Dimethylamine enthaltenden Gemischen.

**Claims**

**1.** A process for the preparation of N,N-dimethylamines by reaction of an aldehyde with dimethylamine and hydrogen under pressure and at elevated temperature in the presence of a hydrogenation catalyst, which comprises separating off unconverted dimethylamine, then adding 0.1 to 25% by weight of formaldehyde or a formaldehyde-forming substance, relative to N,N-dimethylamine, and distilling the mixture.

**2.** The process as claimed in claim 1, wherein the aldehyde is reacted with dimethylamine and hydrogen in a molar ratio of 1 : (1 to 10) : (1 to 50), in particular 1 : (1 to 8) : (1 to 30) at 1 to 20, in particular 3 to 15, MPa and 50 to 150, in particular 70 to 130, °C.

**3.** The process as claimed in claim 1 or 2, wherein the hydrogenation catalyst is a support-free metal catalyst or a supported catalyst containing $Al_2O_3$, $SiO_2$, siliceous earth, activated carbon or pumice, in particular $SiO_2$, siliceous earth or activated carbon, as support.

**4.** The process as claimed in one or more of claims 1 to 3, wherein the hydrogenation catalyst contains Ni, Co, Cu, Mn, Fe, Rh, Pd and/or Pt, in particular Ni, Co, Cu, Rh, Pd and/or Pt and in addition, if desired, customary additives and promotors, such as alkaline earth metal oxides, $SiO_2$, $Al_2O_3$, $MnO_2$ and/or $Cr_2O_3$.

**5.** The process as claimed in one or more of claims 1 to 4, wherein unconverted dimethylamine is separated off by distillation first at 0.5 to 1.0 MPa and then at 0.05 to 0.15 MPa.

**6.** The process as claimed in one or more of claims 1 to 5, wherein 1 to 10, in particular 1.1 to 5, % by weight of formaldehyde or a formaldehyde-forming substance, relative to N,N-dimethylamine, are added.

**7.** The process as claimed in one or more of claims 1 to 6, wherein formaldehyde is added at 0.05 to 1.0, in particular 0.1 to 0.5, MPa and 50 to 250, in particular 70 to 200, °C.

8. The process as claimed in one or more of claims 1 to 7, wherein the distillation is carried out in the presence of a water-entraining solvent and, if necessary, in several steps.

9. Use of formaldehyde or a formaldehyde-forming substance for the removal of secondary N-methylamines from mixtures containing secondary N-methylamines and N,N-dimethylamine.

**Revendications**

1. Procédé pour la préparation de N,N-diméthylamines par réaction d'un aldéhyde avec la diméthylamine et l'hydrogène sous pression et à chaud en présence d'un catalyseur d'hydrogénation, caractérisé en ce que l'on sépare la diméthylamine non convertie, on ajoute ensuite 0,1 à 25 % en poids de formaldéhyde ou d'une substance libérant du formaldéhyde par rapport à la N,N-diméthylamine, et on distille.

2. Procédé selon la revendication 1, caractérisé en ce que l'on fait réagir l'aldéhyde avec la diméthylamine et l'hydrogène dans des proportions molaires de 1 : 1 à 10 : 1 à 50, plus spécialement de 1 : 1 à 8 : 1 à 30, à des pressions de 1 à 20, plus spécialement de 3 à 15 MPa et des températures de 50 à 150, plus spécialement de 70 à 130°C.

3. Procédé selon les revendications 1 ou 2, caractérisé en ce que le catalyseur d'hydrogénation est un catalyseur métallique sans support ou un catalyseur sur support dont le support consiste en $Al_2O_3$, $SiO_2$, alumine, charbon actif ou ponce, plus spécialement $SiO_2$, alumine ou charbon actif.

4. Procédé selon une ou plusieurs des revendications qui précèdent, caractérisé en ce que le catalyseur d'hydrogénation contient Ni, Co, Cu, Mn, Fe, Rh, Pd et/ou Pt, plus spécialement Ni, Co, Cu, Rh, Pd et/ou Pt et le cas échéant des additifs et activateurs usuels tels que des oxydes de métaux alcalino-terreux, $SiO_2$, $Al_2O_3$, $MnO_2$ et/ou $Cr_2O_3$.

5. Procédé selon une ou plusieurs des revendications 1 à 4, caractérisé en ce que l'on sépare la diméthylamine non convertie par distillation, d'abord à une pression de 0,5 à 1,0 MPa, puis à une pression de 0,05 à 0,15 MPa.

6. Procédé selon une ou plusieurs des revendications 1 à 5, caractérisé en ce que l'on ajoute de 1 à 10, plus spécialement de 1,1 à 5 % en poids de formaldéhyde ou d'une substance libérant du formaldéhyde, par rapport à la N,N-diméthylamine.

7. Procédé selon une ou plusieurs des revendications 1 à 6, caractérisé en ce que l'on ajoute le formaldéhyde à des pressions de 0,05 à 1,0, plus spécialement de 0,1 à 0,5 MPa et des températures de 50 à 250, plus spécialement de 70 à 200°C.

8. Procédé selon une ou plusieurs des revendications 1 à 7, caractérisé en ce que l'on distille en présence d'un solvant entraînant l'eau et le cas échéant en plusieurs stades opératoires.

9. Utilisation du formaldéhyde ou d'une substance libérant du formaldéhyde pour éliminer les N-méthylamines secondaires de mélanges contenant des N-méthylamines secondaires et des N,N-diméthylamines.